# EUROPÄISCHE PATENTSCHRIFT

(11) **EP 2 398 430 B1**
(45) Veröffentlichungstag und Bekanntmachung des Hinweises auf die Patenterteilung: **24.09.2014**
(21) Anmeldenummer: 10708153.1
(22) Anmeldetag: 16.02.2010
(51) Int. Cl.: A61F 5/41

(54) **Befestigungsvorrichtung für ein Penisextensionsgerät**
Fastening device for a penis extension device
Dispositif de fixation pour un appareil d'extension de pénis

(30) Priorität: 19.02.2009 EP 09002330
(43) Veröffentlichungstag der Anmeldung: 28.12.2011
(73) Patentinhaber: Swiss Sana Anstalt, 9490 Vaduz (LI)
(72) Erfinder: JOCHUM, Herbert, 82541 Ammerland (DE)
(74) Vertreter: Köster, Hajo
(86) Internationale Anmeldenummer: PCT/EP2010/051908
(87) Internationale Veröffentlichungsnummer: WO 2010/094677

(56) Entgegenhaltungen:
- WO-A-91/17727
- US-A- 5 836 864
- US-A1- 2004 077 970
- US-A1- 2007 093 687
- US-B1- 6 458 073

## Beschreibung

Die Erfindung betrifft eine Befestigungsvorrichtung für den distalen Teil des Penis an einer Penisextensionsvorrichtung, die mit der Befestigungsvorrichtung verbindbar ist und einen Zug auf den Penis ausüben kann, wobei die Befestigungsvorrichtung einen ersten, am proximalen Ende offenen, länglichen, starren Hohlkörper zur Aufnahme des distalen Teil des Penis besitzt und der erste Hohlkörper am proximalen Ende mit einer elastischen Manschette zur Anlage an den Penisschaft ausgestattet ist.

Es sind die verschiedensten Penisextensionsgeräte bekannt, welche eine langzeitige Dehnungsbehandlung des Penis ermöglichen und zu einer Vergrößerung des männlichen Gliedes und der Eichel führen.

Ein typischer Vertreter eines derartigen Penisextensionsgerätes ist in der EP 1 023 013 B1 beschrieben. Weitere derartige Geräte sind in der Beschreibung dieser europäischen Patentschrift aufgeführt.

Bei allen diesen Geräten tritt das Problem auf, dass auf das distale Ende des Penis bzw. die Eichel eine Kraft übertragen werden muss, damit der Penis gedehnt werden kann. Bei dem Penisextensionsgerät gemäß der EP 1 023 013 B1 erfolgt dies mit Hilfe einer Schlaufe.

Es ist auch schon vorgeschlagen worden, für diesen Zweck einen so genannten Eichelschlitten zur Anwendung zu bringen. Diesbezüglich wird verwiesen auf DE 20 2007 003 824 U1 und DE 20 2006 017 667 U1.

Bei den unter der Bezeichnung "Phallosan" vertriebenen Penisextensionsvorrichtung wird ein spezielles Kondom eingesetzt, mit dessen Hilfe der Zug auf den Penis übertragen wird. Dabei entsteht innerhalb des Kondoms ein Vakuum. Bei dieser bekannten Vorrichtung müssen je nach Penisgröße unterschiedlich dimensionierte Kondome eingesetzt werden.

Eine Penisextensionsvorrichtung, bei der eine Befestigungsvorrichtung mit Hilfe von Vakuum mit dem Penis verbunden wird, ist in der EP 1 779 822 B1 (entspricht US 2007/0093687 A1) beschrieben. Weitere Vertreter dieser Art sind in der Beschreibungseinleitung dieser europäischen Patentschrift erwähnt.

Ferner beschreibt die US-A-5 836 864 eine Vorrichtung aus einem Zylinder und einem in diesem Zylinder beweglichen Kolben. Zur Abdichtung des in ein Ende des Zylinders eingeführten Penis dient eine Hülle oder ähnliches. Durch Bewegen des Zylinders in dem Zylinder wird innerhalb des Zylinders ein Vakuum erzeugt. Der Zylinder wird in der gewünschten Position fixiert.

Es sind zudem seit langem zahlreiche Vorrichtungen zur Anwendung von Vakuum auf den Penis zur Erektionsförderung bekannt. So ist in der WO91/17727 A eine Vorrichtung mit einem länglichen Zylinder zur Aufnahme eines Penis und mit einer Vakuumpumpe bekannt. Durch die Betätigung der Pumpe wird innerhalb des Zylinders ein Vakuum erzeugt, um die Peniserektion zu unterstützen.

Eine weitere derartige Vorrichtung zur Behandlung des erektilen Dysfunktion ist aus der US 6 458 073 B1 bekannt, bei der Luft mit Hilfe einer Pumpvorrichtung aus einer länglichen, den Penis aufnehmenden Vakuumkammer herausgesaugt wird.

Die beiden letztgenannten Vorrichtungen dienen jedoch nicht zur Befestigung einer Penisextensionsvorrichtung am Penis, um auf letzteren eine Zugkraft und damit eine Dehnungsbehandlung auszuüben. Daher sind sie keine gattungsgemäßen Vorrichtungen.

Aufgabe der vorliegenden Erfindung ist es, eine verbesserte Befestigungsvorrichtung für gattungsgemäße Penisextensionsvorrichtungen bereitzustellen, deren Anwendung einfacher, wirksamer und schonender ist als die bisher bekannten Befestigungsvorrichtungen.

Gelöst wird diese Aufgabe durch die Lehre der Ansprüche.

Die erfindungsgemäße Befestigungsvorrichtung besitzt ebenso wie die Befestigungsvorrichtung bzw. Zugvakuumvorrichtung gemäß der EP 1 779 822 B1 (entspricht US 2007/0093687 A1) eine starren Hohlkörper zur Aufnahme des distalen Teils des Penis. Mit dem Merkmal "starr" soll zum Ausdruck gebracht werden, dass dieser Hohlkörper im Gegensatz zu einem Kondom formstabil ist und unter normalen Umständen seine Form beibehält.

Die Merkmale "distal" und "proximal" beziehen sich auf denjenigen Zustand, bei dem der Penis in die Befestigungsvorrichtung eingeschoben ist. Diese Ausdrücke werden benutzt, um die beiden Enden der länglichen Hohlkörper einfacher beschreiben zu können bzw. sich einfacher darauf beziehen zu können. Der erste Hohlkörper ist an seinem proximalen Ende mit einer elastischen Manschette versehen, welche den ersten Hohlkörper mit dem Penis bzw. dem Penisschaft verbindet.

Bei einer derartigen elastischen Manschette kann es sich im einfachsten Fall um einen Kondom handeln, dessen geschlossenes Ende abgeschnitten ist und das auf die Außenmantelfläche des ersten Hohlkörpers aufgeschoben bzw. aufgerollt ist. Nach Einführen des Penis in diesen Hohlkörper wird das Kondom einfach nur teilweise abgerollt und in Anlage an den Penisschaft gebracht. Dadurch wird dieses Ende des Hohlköpers abgedichtet.

Dieser erste Hohlkörper ist jedoch nicht nur am proximalen Ende sondern auch am distalen Ende geöffnet.

Neben diesem ersten Hohlkörper ist bei der erfindungsgemäßen Befestigungsvorrichtung auch ein zweiter starrer Hohlkörper vorhanden, in den der erste Hohlkörper hineinragt. Dieser zweite Hohlkörper ist am proximalen Ende offen sowie am distalen Ende geschlossen.

Bei beiden Hohlkörpern handelt es sich vorzugsweise um rohrförmige Elemente und weiterhin bevorzugt um zylinderrohrförmige Elemente. Das den zweiten Hohlkörper bildende rohrförmige Element ist am distalen Ende geschlossen. Das den ersten Hohlkörper im wesentlichen ausmachende rohrförmige Element ist am distalen Ende vorzugsweise kuppelförmig ausgebildet.. Dadurch wird die Eichel des Penis geschützt. Diese Kuppel besitzt mindestens eine durchgehende Öffnung.

Der erste Hohlkörper ist in den zweiten Hohlkörper in der Art eines Kolbens hin und her bewegbar. Mit diesem Merkmal soll zum Ausdruck gebracht werden, dass die beiden Hohlkörper in Längsrichtung zueinander verschiebbar sind, jedoch eine Abdichtung zwischen der Außenmantelfläche des ersten Hohlkörpers und der gegenüberliegenden Innenmantelfläche des zweiten Hohlkörpers gegeben ist.

Die beiden Hohlkörper können im Prinzip jede beliebige Querschnittsform besitzen, beispielsweise rechteckig, viereckig und rund. Erforderlich ist lediglich, die Querschnittsformen der beiden Hohlkörper flächenkongruent sind, so dass der erste Hohlkörper in der Art eines Kolbens im bzw. innerhalb des zweiten Hohlkörper in abdichtender Weise hin- und herbewegbar ist. Wie bei einem Kolben in einem Zylinder soll die Außenmantelfläche des ersten Hohlkörpers von der Innenmantelfläche des zweiten Hohlkörpers möglichst wenig beabstandet sein, sodass die gewünschte Abdichtung erzielt wird. Gewünschtenfalls kann ein dichtender O-Ring oder ähnliches eingesetzt werden.

Diese Abdichtung ist erforderlich, um in der erfindungsgemäßen Befestigungsvorrichtung ein Vakuum erzeugen zu können. Ist nämlich die Manschette mit dem ersten Hohlkörper sowie dem Penis verbunden, ist dieses Ende der erfindungsgemäßen Befestigungsvorrichtung verschlossen. Das anderer Ende ist durch das verschlossene Ende des zweiten Hohlkörpers verschlossen. Werden die beiden Hohlkörper nun derart bewegt, dass der erste Hohlkörper aus dem zweiten Hohlkörper herausgezogen wird, dann entsteht innerhalb der Gesamtheit aus erstem und zweitem Hohlkörper ein Unterdruck.

Das Vakuum innerhalb des von den beiden Hohlkörpern sowie der Manschette begrenzten Innenraumes wird somit lediglich durch ein Gegeneinander-Verschieben der beiden Hohlkörper erzeugt. Weitere zusätzliche Einrichtungen sind für die Erzeugung eines Vakuums nicht erforderlich. Daher werden die Innenräume der beiden Hohlkörper auch nicht durch weitere Einrichtungen oder Teile eingeschränkt.

Das Vakuum dient auch nicht primär als Mittel zur Beeinflussung oder Behandlung des Penis. Vielmehr soll das Vakuum dafür Sorge tragen, dass die durch das Penisextensionsgerät ausgeübte Zugkraft auf den Penis übertragen wird und für seine Dehnung bzw. Streckung Sorge trägt.

Der Penis befindet sich im Inneren des ersten Hohlkörpers und wird durch diesen geschützt. Beim Hineinschieben des ersten Hohlkörpers in den zweiten Hohlkörper bleibt dieser Schutz erhalten.

Zum Anlegen der erfindungsgemäßen Befestigungsvorrichtung kann man beispielsweise wie folgt vorgehen. Man schiebt den ersten Hohlkörper soweit wie möglich in den zweiten Hohlkörper hinein und verbindet dann die Manschette mit dem Penisschaft. Beim Auseinanderziehen der beiden Hohlkörper entsteht dann ein Vakuum.

Nach einer bevorzugten Ausführungsform ist der zweite Hohlkörper an seinem distalen Ende mit einer verschließbaren Öffnung versehen. Dies hat den Vorteil, dass man erst einmal den Penis in den ersten Hohlkörper einführen und die Manschette anlegen kann. Anschließend schiebt man den zweiten Hohlkörper auf den ersten Hohlkörper auf, wobei die Öffnung geöffnet ist, so dass Luft aus dem zweiten Hohlkörper entweichen kann. Anschließend verschließt man die Öffnung und damit auch den zweiten Hohlkörper.

Natürlich ist es auch möglich, den ersten Hohlkörper in den zweiten Hohlkörper einzusetzen und dann den Penis in den ersten Hohlkörper einzuführen und die Manschette auf dem Penisschaft anzubringen.

Nach einer weiterhin bevorzugten Ausführungsform ist der erste Hohlkörper zwischen zwei Endpunkten im zweiten Hohlkörper hin- und her bewegbar. Um dies zu gewährleisten, besitzt der erste Hohlkörper vorzugsweise außen eine sich in Längsrichtung zwischen zwei Endpunkten erstreckende Führungsnut, in die ein innen am zweiten Hohlkörper angebrachter Führungszapfen eingreift. Dieser Führungszapfen ist vorzugsweise am proximalen Rand des zweiten Hohlkörpers angeordnet.

Die Führungsnut des ersten Hohlkörpers geht vorzugsweise am distalen Endpunkt in einen sich quer zur Längsrichtung erstreckenden Nutabschnitt über. Dieser Nutabschnitt mündet an einem Ende in die sich in Längsrichtung erstreckende Führungsnut und ist am anderen Ende geschlossen. Wird der Führungszapfen in diesem Nutabschnitt bewegt, dann ist die axiale Verschiebung der beiden Hohlkörper zueinander blockiert bzw. nicht mehr möglich. Auf diese Weise kann eine Arretierung erfolgen.

Die Manschette, welche zur Verbindung des ersten Hohlkörpers mit dem Penisschaft dient, ist vorzugsweise an einem Ende mit einem steifen Ring (vorzugsweise Zylinderring) versehen, der auf das proximale Ende oder in das proximale Ende des ersten Hohlkörpers unter Herstellung einer reibschlüssigen oder formschlüssigen, jedoch lösbaren Verbindung auf- oder einsetzbar ist. Mit dieser Ausführungsform ist es möglich, zuerst die Manschette mit dem Penisschaft zu verbinden. Dazu wird der Penis in den Ring eingeführt, die Manschette kann dabei aufgerollt sein. Danach wird die Manschette abgerollt und stellt die abdichtende Verbindung her. Anschließend wird dann der Ring mit dem ersten Hohlkörper verbunden, wobei dieser erste Hohlkörper sowohl bereits in den zweiten Hohlkörper eingeschoben als auch noch nicht eingeschoben sein kann. Auch die umgekehrte Reihenfolge der Handhabung kann gewählt werden.

Die verschließbare Öffnung des zweiten Hohlkörpers stellt vorzugsweise ein manuell betätigbares Ventil oder ein Wegeventil dar, dass eine Luftstrom nur nach außen zulässt. Wird beispielsweise der erste Hohlkörper mit darin eingeführtem Penis in den zweiten Hohlkörper eingeschoben, dann kann durch das manuell geöffnete Ventil oder das Wegeventil die durch Zusammenschieben der beiden Hohlkörper komprimierte Luft entweichen. Werden die beiden Hohlkörper dann anschließend auseinander gezogen, dann entsteht bei geschlossenem Ventil keine Möglichkeit mehr, dass die Luft zurück strömt. Auf diese Weise wird ein Vakuum erzeugt.

Vorzugsweise besitzt der zweite Hohlkörper an seinem distalen Ende eine dam i t verbindbare und auch wieder lösbare Verschlusseinrichtung. Der zweite Hohlkörper weist somit zwei verbindbare und auch wieder lösbare Teile auf. Bei dieser Verschlussvorrichtung handelt es sich vorzugsweise um eine Kappe oder einen Deckel, die bzw. der auf das oder in das distale Ende des zweiten Hohlkörpers aufschraubbar oder einschraubbar ist. In dieser separaten Verschlusseinrichtung ist vorzugsweise die verschließbare Öffnung ausgebildet.

Bedingt dadurch, dass die Manschette vorzugsweise mit dem oben geschilderten starren Ring ausgestattet ist, kann der erste Hohlkörper und somit auch die erfindungsgemäße Befestigungsvorrichtung mit unterschiedlich dimensionierten Manschetten verbunden werden, um den verschiedenen Penisgrößen Rechnung zu tragen.

Die Erfindung wird im folgenden anhand der beiliegenden Zeichnungen, welche die erfindungsgemäße Befestigungsvorrichtung skizzenhaft und nicht maßstabsgetreuer weise zeigen, näher erläutert.

In den Zeichnungen zeigen:
- Fig. 1: eine Längsschnittansicht einer erfindungsgemäßen Befestigungsvorrichtung mit in den ersten Hohlkörper eingeführten Penis und mit maximal in den zweiten Hohlkörper eingeschobenen ersten Hohlkörper,
- Fig. 2: eine der Fig. 1 analoge Längsschnittansicht, bei der sich die beiden Hohlkörper in maximal auseinander gezogenem Zustand befinden,
- Fig. 3: die Einzelteile der in den Figuren 1 und 2 gezeigten Befestigungsvorrichtung in Seitenansicht (teilweise weggebrochen),
- Fig. 4: eine der Fig. 2 analoge Ansicht einer weiteren Ausführungsform einer erfindungsgemäßen Befestigungsvorrichtung und
- Fig. 5.: eine der Fig. 3 analoge Ansicht der in der Fig. 4 gezeigten bevorzugten Ausführungsform.

Die erfindungsgemäße Befestigungsvorrichtung 1 besitzt einen ersten Hohlkörper 2 und eine zweiten Hohlkörper 3.

Beide Hohlkörper 2 und 3 sind am proximalen Ende (links in den Figuren) offen und sind länglich ausgestaltet sowie starr. Diese Hohlkörper 2 und 3 können aus jedem geeigneten Material bestehen, beispielsweise Kunststoff, Glas, Gummi, Metall oder Keramik. Vorzugsweise sind sie aus einem Kunststoff gefertigt.

Der erste Hohlkörper 2 stellt vorzugsweise eine Zylinderhülse bzw. ein Zylinderrohr dar. Der zweite Hohlkörper 3 besitzt einen zylinderrohrförmigen Abschnitt 5, der am distalen Ende in eine Kappe 4 übergeht.

Der zylinderrohrförmige erste Hohlkörper 2 besitzt an seinem proximalen Ende einen Zylinderrohrabschnitt 6, dessen Außendurchmesser geringer ist als der des ersten Hohlkörpers 2. Die Innendurchmesser sind gleich. Am Übergang vom zylinderrohrförmigen Abschnitt 6 zum ersten Hohlkörper 2 entsteht dadurch ein Absatz 7.

Auf diesen Zylinderrohrabschnitt 6 ist ein Zylinderring 8 aufschiebbar und dadurch mit dem ersten Hohlkörper 2 verbindbar. Der Zylinderring 8 ist ebenfalls aus einem starren Material gefertigt, beispielsweise Kunststoff. Am proximalen Ende des Zylinderrings 8 (in den Figuren links) ist eine elastische Manschette 9 angebracht, die aus einem gummiartigen Material entsprechend einem Kondom gefertigt ist.

Der zweite Hohlkörper 3 besitzt an seinem distalen Ende und somit in der Kappe 4 ein Wegeventil 10, das mit einer Öffnung 11 ausgestattet ist, die mit einem elastischen scheibenartigen Plättchen 12 verschlossen ist. Dieses Plättchen 12 liegt in proximaler Richtung auf einem umlaufenden Ringabsatz 13 auf und ist dort abgestützt. Ferner ist es zentrisch abgestützt. Die beiden Hohlkörper 2 und 3 sind derart dimensioniert, dass der erste Hohlkörper 2 in den Innenraum 14 des zweiten Hohlkörpers 3 eingeführt werden kann und eine Hin- und Herbewegung in der Richtung der Längsachse 15 ausführen kann. Die beiden Längsachsen 15 der beiden Hohlkörper fallen im übrigen zusammen.

Ferner gleitet die Außenmantelfläche des ersten Hohlkörpers 2 an der Innenmantelfläche des zylinderrohrförmigen Abschnitts 5 des zweiten Hohlkörpers 3 in möglichst dichtem Abstand entlang, damit eine Abdichtung des Innenraum 14 und auch des Innenraums 16 des ersten Hohlkörpers 2 erreicht wird. Die weitere Abdichtung dieser miteinander kommunizierenden Innenräume 14, 16 erfolgt zum proximalen Ende hin durch die Manschette 9 und zum distalen Ende hin durch die Kappe 4 bzw. das Wegeventil 10, dass eine Bewegung des in den Innenräumen 14, 16 befindlichen Fluids (hier Luft) nur nach außen hin ermöglicht und ein zurückfließen der Luft verhindert.

Die einander gegenüberliegenden Mantelflächen des ersten und zweiten Hohlkörpers 2, 3 können auch mit einem Gleitmittel oder Ähnlichem beschichtet sein, um eine bessere Abdichtung zu erzielen.

Der erste Hohlkörper 2 ist mit einer nach außen offenen, sich in Richtung der Längsachse 15 erstreckenden, an ihren Enden geschlossenen Führungsnut 17 versehen, die an ihrem distalen Ende bzw. Endpunkt in einen sich quer zur Längsrichtung bzw. Längsachse 15 erstreckenden Nutabschnitt 18 übergeht. Der an seinem anderen Ende geschlossen ist.

In diese Führungsnut 17 greift ein Führungszapfen 19 ein, der am proximalen Rand innen am zweiten Hohlkörper 3 ausgebildet ist. Die beiden Hohlkörper 2, 3 die relativ zueinander verschiebbar sind, können somit nur über eine bestimmte Wegstrecke in Längsrichtung verschoben werden. Mit anderen Worten, der erste Hohlkörper 2 kann bezüglich des zweiten Hohlkörpers 3 eine Translationsbewegung in Längsrichtung zwischen den beiden Eckpunkten bzw. Enden der Führungsnut 17 ausführen.

Zum Verriegeln der beiden Hohlkörper 2, 3 werden diese zueinander verdreht, so dass der Führungszapfen 19 in dem Nutabschnitt 18 zu liegen kommt. In diesem Zustand ist keine Relativbewegung der beiden Hohlkörper 2, 3 in Axialrichtung mehr möglich.

Der zweite Hohlkörper 3 besitzt in der Nähe seines proximalen Endes zwei diametral einander gegenüberliegende, nach radial außen ragende Noppen 20, an denen ein Benutzer den zweiten Hohlkörper 3 ergreifen und mit deren Hilfe er diesen verschieben kann.

Zum Anbringen der erfindungsgemäßem Befestigung zur Vorrichtung geht ein Benutzer beispielsweise wie folgt vor.

Als erstes wählt der Benutzer eine Manschette 9 aus, welche der Größe seines Penis Rechnung trägt.

Diese Manschette 9 setzt der Benutzer mit Hilfe des Zylinderrings 8 auf den Zylinderrohrabschnitt 6 der ersten Hohlkörpers 2 auf und stellt eine Verbindung dieser beiden Teile her. Der erste Hohlkörper 2 ist übrigens schon in den zweiten Hohlkörper 3 eingesetzt, so dass der Führungszapfen 19 in die Führungsnut 17 eingreift.

Nachdem der Benutzer die Manschette 9 mittels des Zylinderringes 8 mit dem ersten Hohlkörper 2 verbunden hat, trägt er dafür Sorge, dass die Manschette 9 auf den zweiten Hohlkörper 2 aufgerollt ist.

Anschließend führt der Benutzer seinen Penis in den ersten Hohlkörper 2 ein und rollt die elastische Manschette 9 auf den Penisschaft ab. Dadurch wird die Position des ersten Hohlkörpers 2 bezüglich des Penis festgelegt. Anschließend schiebt der Benutzer den zweiten Hohlkörper 3 in proximale Richtung, und zwar so lange, bis das Volumen des Innenraums 14 möglichst gering ist. Dabei wird die in diesem Innenraum 14 befindliche Luft komprimiert und kann durch das sich öffnende Wegeventil 10 nach außen heraus strömen.

Wird danach der zweite Hohlkörper 3 bei geschlossenem Wegeventil 10 in distale Richtung bezüglich des ersten Hohlkörpers 2 verschoben, dann entsteht im Innenraum 14 bzw. im Innenraum 16 ein Vakuum. Dieses Vakuum ist umso größer, je mehr die beiden Hohlräume 2, 3 voneinander weg bewegt werden.

Wichtig dabei ist, dass innerhalb der Kombination aus den Hohlkörpern 2 und 3 ein Vakuum erzeugt werden kann, ohne dass es zu einer ausprägten Relativbewegung zwischen dem Penis bzw. dem Penisschaft und dem ersten Hohlkörper 2 kommt. Auf diese Weise kann eine Reizung des Penis vermieden werden.

Wird die Position der beiden Hohlkörper 2, 3 zueinander fixiert, indem der Führungszapfen 19 in dem Nutabschnitt 18 zu liegen kommt, dann hat das in den Innenräumen 14, 15 herrschende Vakuum seinen Maximalwert. Zudem ändert sich dieses Vakuum nicht, da keine Relativbewegung der beiden Hohlkörper 2, 3 mehr erfolgen kann.

Um die erfindungsgemäße Befestigungsvorrichtung 1 mit einer Penisextensionsvorrichtung zu verbinden, welche einen Zug auf diese Befestigungsvorrichtung 1 und somit auch auf den Penis ausübt, kann die Befestigungsvorrichtung 1 mit einer Zugvorrichtung ausgestattet sein, wobei es sich beispielsweise um einen Haken, eine Öse, ein Band oder ähnliches handeln kann. Diese Zugvorrichtung wird an dem weiten Hohlkörper 3 befestigt oder ist damit fest verbunden und ist üblicher Art und daher in den Figuren nicht dargestellt.

Die Kappe 4 des zweiten Hohlkörpers 3 kann im übrigen derart ausgestaltet sein, dass sie mit dem zweiten Hohlkörper 3 durch Verschrauben etc. verbindbar ist (in den Figuren nicht dargestellt). Dadurch wird bei abgeschraubter Kappe ein Urinieren ermöglicht, ohne die Befestigungsvorrichtung entfernen zu müssen.

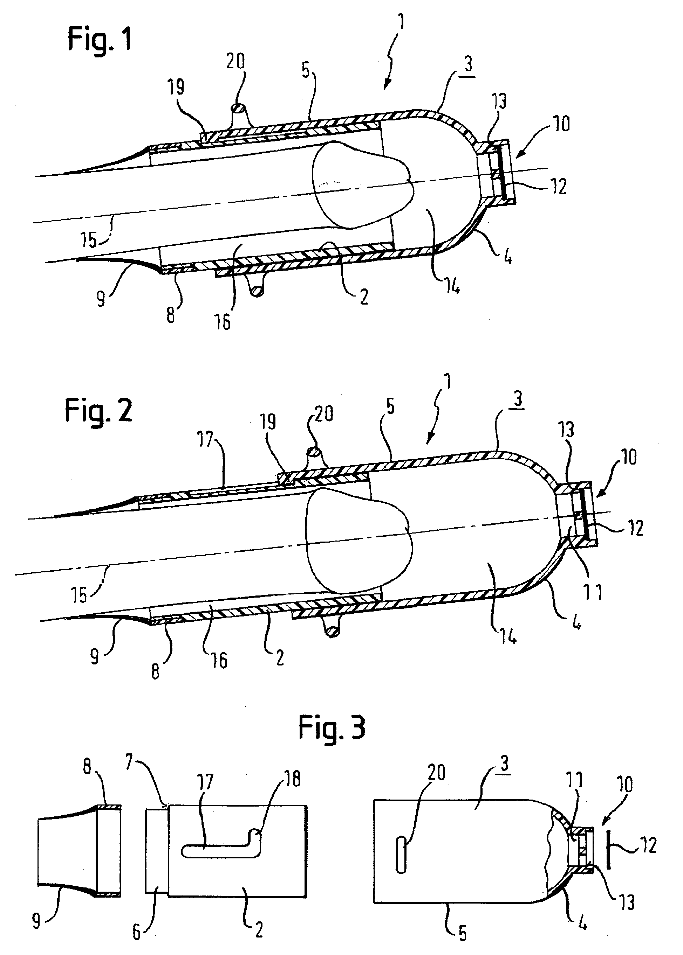

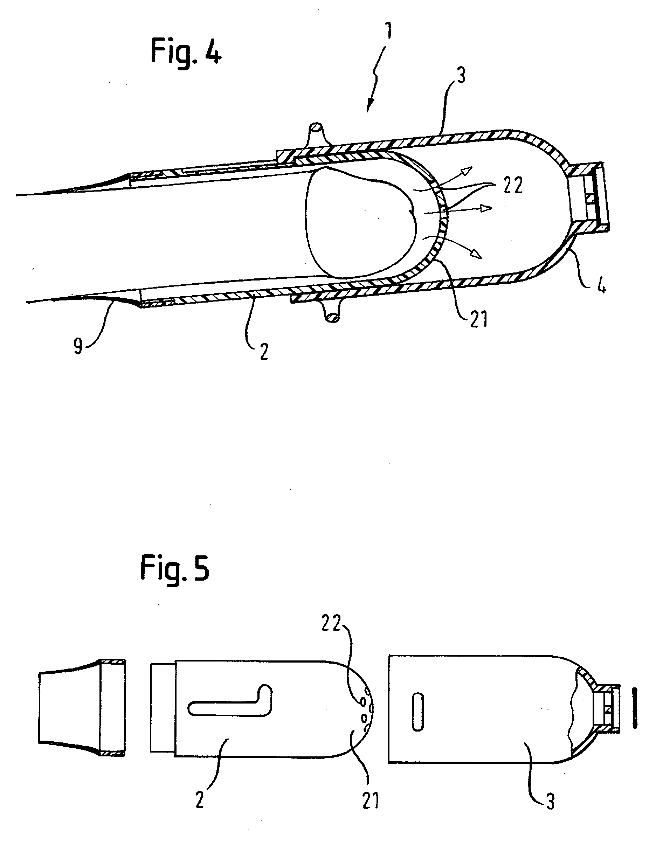

Die in den Figuren 4 und 5 gezeigte Befestigungsvorrichtung 1 unterscheidet sich von der in den Figuren 1-3 gezeigten Befestigungsvorrichtung 1 lediglich dadurch, dass der zylinderrohrförmige erste Hohlkörper 2 an seinem distalen Ende durch eine Kuppel 21 abgeschlossen wird, die mehrere durchgehende Öffnungen 22 aufweist.

Diese Ausführungsform hat folgenden Vorteil. Bei Gebrauch der erfindungsgemäßen Vorrichtung wirkt auf den Penis und somit auch auf die Eichel ein Unterdruck, wodurch eine Volumenvergrößerung in axialer und auch in radialer Richtung erfolgt. Der Penis bzw. die Eichel kommt dabei in Anlage an die Innenmantelfläche des ersten Hohlkörpers 2, wodurch eine weitere Volumenvergrößerung verhindert wird.

### Bezugszeichenliste

- 1.: Befestigungsvorrichtung
- 2.: erster Hohlkörper
- 3.: zweiter Hohlkörper
- 4.: Kappe
- 5.: zylinderrohrförmiger Abschnitt
- 6.: Zylinderrohrabschnitt
- 7.: Absatz
- 8.: Zylinderring
- 9.: Manschette
- 10.: Wegeventil
- 11.: Öffnung
- 12.: scheibenartiges Plättchen
- 13.: Ring
- 14.: Innenraum
- 15.: Längsachse
- 16.: Innenraum
- 17.: Führungsnut
- 18.: Nutabschnitt
- 19.: Führungszapfen
- 20.: Noppen
- 21.: Kuppel
- 22.: Öffnung

## Patentansprüche

1. Befestigungsvorrichtung (1) für den distalen Teil des Penis an einer Penisextensionsvorrichtung, die mit der Befestigungsvorrichtung (1) verbindbar ist und einen Zug auf den Penis ausüben kann, wobei die Befestigungsvorrichtung (1) einen ersten, am proximalen Ende offenen, länglichen, starren Hohlkörper (2) zur Aufnahme des distalen Teils des Penis besitzt und der erste Hohlkörper (2) am proximalen Ende mit einer elastischen Manschette (9) zur Anlage an den Penisschaft ausgestattet ist,
**dadurch gekennzeichnet, dass**
der erste Hohlkörper (2) auch am distalen Ende offen ist und in einen zweiten, am proximalen Ende offenen, am distalen Ende geschlossenen, länglichen, starren Hohlkörper (3) hineinragt, und dass die Querschnittsformen der beiden Hohlkörper (2, 3) flächenkongruent sind, so dass der erste Hohlkörper (2) in der Art eines Kolbens im zweiten Hohlkörper (3) in abdichtender Weise hin- und herbewegbar ist.

2. Befestigungsvorrichtung nach Anspruch 1,
**dadurch gekennzeichnet, dass**
beide Hohlkörper (2, 3) im wesentlichen zylinderrohrförmig sind.

3. Befestigungsvorrichtung nach Anspruch 2,
**dadurch gekennzeichnet, dass**
der zweite Hohlkörper (3) an seinem distalen Ende kuppelförmig ausgebildet ist.

4. Befestigungsvorrichtung nach einem der vorhergehenden Ansprüche,
**dadurch gekennzeichnet, dass**
der erste Hohlkörper (2) zwischen zwei Endpunkten im zweiten Hohlkörper (3) hin- und herbewegbar ist.

5. Befestigungsvorrichtung nach Anspruch 4,
**dadurch gekennzeichnet, dass**
der erste Hohlkörper (2) außen eine sich in Längsrichtung zwischen zwei Endpunkten erstreckende Führungsnut (19) besitzt, in die ein innen am zweiten Hohlkörper (3) angebrachter Führungszapfen eingreift.

6. Befestigungsvorrichtung nach Anspruch 5,
**dadurch gekennzeichnet, dass**
sich der Führungszapfen (19) am proximalen Rand des zweiten Hohlkörpers (3) befindet.

7. Befestigungsvorrichtung nach Anspruch 5 oder 6,
**dadurch gekennzeichnet, dass**
die Führungsnut (17) des ersten Hohlkörper (2) am distalen Endpunkt in einen sich quer zur Längsrichtung erstreckenden Nutabschnitt (18) übergeht.

8. Befestigungsvorrichtung nach einem der vorhergehenden Ansprüche,
**dadurch gekennzeichnet, dass**
die Manschette (9) an einem Ende mit einem steifen Ring, insbesondere Zylinderring (8) versehen ist, der auf oder in das proximale Ende des ersten Hohlkörpers (2) unter Herstellung einer reibschlüssigen oder formschlüssigen, jedoch lösbaren Verbindung auf- oder einsetzbar ist.

9. Befestigungsvorrichtung nach einem der vorhergehenden Ansprüche,
**dadurch gekennzeichnet, dass**
der zweite Hohlkörper (3) an seinem distalen Ende eine damit verbindbare und auch wieder lösbare Verschlusseinrichtung besitzt

10. Befestigungsvorrichtung nach Anspruch 9,
**dadurch gekennzeichnet, dass**
die Verschlusseinrichtung eine Kappe (4) oder einen Deckel darstellt.

11. Befestigungsvorrichtung nach Anspruch 9 oder 10,
**dadurch gekennzeichnet, dass**
die Verschlusseinrichtung auf das oder in das distale Ende des zweiten Hohlkörpers (3) aufschraubbar oder einschraubbar ist.

12. Befestigungsvorrichtung nach einem der vorhergehenden Ansprüche,
**dadurch gekennzeichnet, dass**
der zweite Hohlkörper (3) am distalen Ende mit einer verschließbaren Öffnung (11) versehenen ist.

13. Befestigungsvorrichtung nach Anspruch 12,
**dadurch gekennzeichnet, dass**
die verschließbare Öffnung (11) des zweiten Hohlkörpers (3) ein manuell betätigbares Ventil oder ein Wegeventil (10), das einen Luftstrom nur nach außen zulässt, darstellt.

14. Befestigungsvorrichtung nach Anspruch 12 oder 13,
**dadurch gekennzeichnet, dass**
die verschließbaren Öffnung (11) in der Verschlusseinrichtung ausgebildet ist.

15. Befestigungsvorrichtung nach einem der vorhergehenden Ansprüche,
**dadurch gekennzeichnet, dass**
der erste Hohlkörper (2) an seinem distalen Ende durch eine Wand oder eine Kuppel (21) abgeschlossen wird, die mindestens eine durchgehende Öffnung (22) aufweist.

## Claims

1. Fastening device (1) for the distal part of the penis on a penis extension device which can be connected to the fastening device (1) and can exert traction on the penis, wherein the fastening device (1) has a first oblong rigid hollow body (2) which is open at the proximal end for receiving the distal part of the penis and wherein the first hollow body (2) is provided at the proximal end with an elastic sleeve (9) for bearing against the penis shaft, **characterised in that** the first hollow body (2) is also open at the distal end and projects into a second oblong rigid hollow body (3) which is open at the proximal end and closed at the distal end, and that the cross-sectional shapes of the two hollow bodies (2, 3) have congruent surfaces so that the first hollow body (2) can be moved to and fro in sealing manner like a piston in the second hollow body (3).

2. Fastening device according to claim 1 **characterised in that** both hollow bodies (2, 3) are substantially tubular in design.

3. Fastening device according to claim 2 **characterised in that** the second hollow body (3) is designed dome-shaped at its distal end.

4. Fastening device according to one of the preceding claims **characterised in that** the first hollow body (2) can be moved to and fro between two end points in the second hollow body (3).

5. Fastening device according to claim 4 **characterised in that** the first hollow body (2) has on the outside a guide groove (17) which extends in the longitudinal direction between two end points and into which a guide stud attached on the inside on the second hollow body (3) engages.

6. Fastening device according to claim 5 **characterised in that** the guide stud (19) is located at the proximal edge of the second hollow body (3).

7. Fastening device according to claim 5 or 6 **characterised in that** the guide groove (17) of the first hollow body (2) changes at the distal end point into a groove section (18) extending transversely to the longitudinal direction.

8. Fastening device according to one of the preceding claims **characterised in that** the sleeve (9) is provided at one end with a rigid ring, more particularly a cylindrical ring (8), which can be fitted onto or inserted into the proximal end of the first hollow body (2) to produce a friction-engaging or positive-locking but releasable connection.

9. Fastening device according to one of the preceding claims **characterised in that** the second hollow body (3) has at its distal end a closure device which can be connected thereto but also can be released therefrom.

10. Fastening device according to claim 9 **characterised in that** the closure device has a cap (4) or a cover.

11. Fastening device according to claim 9 or 10 **characterised in that** the closure device can be screwed onto or into the distal end of the second hollow body (3).

12. Fastening device according to one of the preceding claims, **characterised in that** the second hollow body (3) is provided at the distal end with a closable opening (11).

13. Fastening device according to claim 12 **characterised in that** the closable opening (11) of the second hollow body (3) represents a manually operable valve or a directional valve (10) which permits an air flow only to the outside.

14. Fastening device according to claim 12 or 13 **characterised in that** the closable opening (11) is formed in the closure device.

15. Fastening device according to one of the preceding claims **characterised in that** the first hollow body (2) is closed at its distal end by a wall or a dome (21) which has at least one through opening (22).

## Revendications

1. Dispositif de fixation (1) pour la partie distale du pénis sur un dispositif d'extension du pénis qui peut être raccordé au dispositif de fixation (1) et qui peut exercer une traction sur le pénis, le dispositif de fixation (1) possédant un premier corps creux (2) rigide, oblong, ouvert au niveau de l'extrémité proximale pour recevoir la partie distale du pénis, et le premier corps creux (2) étant, au niveau de l'extrémité proximale, muni d'une manchette (9) élastique destinée à venir en appui sur le corps du pénis,
**caractérisé en ce que**
le premier corps creux (2) est ouvert également au niveau de l'extrémité distale et fait saillie dans un deuxième corps creux (3) rigide oblong ouvert au niveau de l'extrémité proximale, fermé au niveau de l'extrémité distale, et **en ce que** les formes de section transversale des deux corps creux (2, 3)
sont congruentes au niveau de leurs surfaces de telle sorte que le premier corps creux (2) peut être déplacé en avant et en arrière de façon étanche à la façon d'un piston dans le deuxième corps creux (3).

2. Dispositif de fixation selon la revendication 1,
**caractérisé en ce que**
les deux corps creux (2, 3) sont essentiellement en forme de tube cylindrique.

3. Dispositif de fixation selon la revendication 2,
**caractérisé en ce que**
le deuxième corps creux (3) est constitué en forme de coupole au niveau de son extrémité distale.

4. Dispositif de fixation selon une des revendications précédentes,
**caractérisé en ce que**
le premier corps creux (2) peut être déplacé en avant et en arrière entre deux points extrêmes dans le deuxième corps creux (3).

5. Dispositif de fixation selon la revendication 4,
**caractérisé en ce que**
le premier corps creux (2) possède à l'extérieur une rainure de guidage (17), s'étendant dans la direction longitudinale entre deux points extrêmes, dans laquelle engrène un ergot de guidage mis en place à l'intérieur sur le deuxième corps creux (3).

6. Dispositif de fixation selon la revendication 5,
**caractérisé en ce que**
l'ergot de guidage (19) est situé sur le bord proximal du deuxième corps creux (3).

7. Dispositif de fixation selon la revendication 5 ou 6,
**caractérisé en ce que**
la rainure de guidage (17) du premier corps creux (2) se transforme, au niveau du point extrême distal, en un segment de rainure (18) s'étendant transversalement à la direction longitudinale.

8. Dispositif de fixation selon une des revendications précédentes,
**caractérisé en ce que**
la manchette (9) est, à une extrémité, munie d'une bague rigide, en particulier une bague cylindrique (8), qui peut être placée sur ou introduite dans l'extrémité proximale du premier corps creux (2) avec réalisation d'un raccordement par liaison de friction ou par liaison de forme qui peut toutefois être détaché.

9. Dispositif de fixation selon une des revendications précédentes,
**caractérisé en ce que**
le deuxième corps creux (3) possède à son extrémité distale un dispositif de fermeture qui peut lui être raccordé et qui peut lui aussi être détaché de nouveau.

10. Dispositif de fixation selon la revendication 9,
**caractérisé en ce que**
le dispositif de fermeture constitue un capuchon (4) ou un couvercle.

11. Dispositif de fixation selon la revendication 9 ou 10,
**caractérisé en ce que**
le dispositif de fermeture peut être vissé sur ou dans l'extrémité distale du deuxième corps creux (3).

12. Dispositif de fixation selon une des revendications précédentes,
**caractérisé en ce que**
le deuxième corps creux (3) est, à l'extrémité distale, muni d'une ouverture (11) pouvant être fermée.

13. Dispositif de fixation selon la revendication 12,
**caractérisé en ce que**
l'ouverture (11) pouvant être fermée du deuxième corps creux (3) constitue un clapet ou un tiroir de distribution (10) pouvant être actionné manuellement qui ne permet un écoulement d'air que vers l'extérieur.

14. Dispositif de fixation selon la revendication 12 ou 13,
**caractérisé en ce que**
l'ouverture (11) pouvant être fermée est formée dans le dispositif de fermeture.

15. Dispositif de fixation selon une des revendications précédentes,
**caractérisé en ce que**
le premier corps creux (2) est, à son extrémité distale, terminé par une paroi ou une coupole (21) qui présente au moins une ouverture débouchante (22).
